# EUROPÄISCHE PATENTSCHRIFT

(11) **EP 3 062 834 B1**
(45) Veröffentlichungstag und Bekanntmachung des Hinweises auf die Patenterteilung: **12.02.2020**
(21) Anmeldenummer: 14792514.3
(22) Anmeldetag: 31.10.2014
(51) Int. Cl.: A61M 1/16

(54) **BLUTBEHANDLUNGSVORRICHTUNG MIT ERHÖHTER PATIENTENSICHERHEIT UND VERFAHREN**
BLOOD TREATMENT DEVICE HAVING INCREASED PATIENT SAFETY AND METHOD
DISPOSITIF DE TRAITEMENT SANGUIN APPORTANT UNE SÉCURITÉ ACCRUE AU PATIENT ET PROCÉDÉ

(30) Priorität: 31.10.2013 DE 102013112038
(43) Veröffentlichungstag der Anmeldung: 07.09.2016
(73) Patentinhaber: Fresenius Medical Care Deutschland GmbH, 61352 Bad Homburg (DE); Fresenius Medical Care AG & Co. KGaA, 61352 Bad Homburg (DE)
(72) Erfinder: WIESEN, Gerhard, 61352 Bad Homburg (DE); POHL, Thomas, 61381 Friedrichsdorf (DE); RAU, Jenspeter, 80801 München (DE)
(74) Vertreter: Bobbert & Partner Patentanwälte PartmbB
(86) Internationale Anmeldenummer: PCT/EP2014/073452
(87) Internationale Veröffentlichungsnummer: WO 2015/063267

(56) Entgegenhaltungen:
- WO-A1-96/09080
- US-A- 4 153 554
- US-A- 4 897 184
- US-A- 5 589 070
- US-A1- 2011 089 112

## Beschreibung

Die vorliegende Erfindung betrifft eine Blutbehandlungsvorrichtung gemäß dem Oberbegriff des Anspruchs 1. Sie betrifft zudem ein Verfahren gemäß dem Oberbegriff des Anspruchs 10 und ein nicht-therapeutisches Verfahren zum Wechseln eines Dialyse- oder Blutfilters einer Blutbehandlungsvorrichtung gemäß dem Oberbegriff des Anspruchs 12.

Aus der Praxis sind Blutbehandlungsvorrichtungen mit Blutfiltern oder Dialysefiltern bekannt. Durch den Dialysefilter werden sowohl Blut als auch Dialysierflüssigkeit in durch eine Dialysemembran getrennt voneinander vorliegenden Kompartments, das Blutkompartment und das Dialysatkompartment, geführt. Der Dialysefilter ist mittels eines extrakorporalen Blutkreislaufs mit dem Patienten als auch mittels einer zuführenden Dialysierflüssigkeitszulaufleitung und einer abführenden Dialysatablaufleitung, welche zur Verbindung mit dem Dialysefilter je einen Verbinder aufweisen, mit der Blutbehandlungsvorrichtung verbunden. Man spricht hierbei von einer Dialysatseite und einer Blutseite.

In äußerst seltenen Fällen kann Blut nach einer Ruptur der Dialysemembran aus dem Blutkompartment in das Dialysatkompartment übertreten. Man spricht dabei von einem Blutleck. Ein solches Blutleck verursacht den Übertritt von Blut in das Dialysatkompartment und damit auf die Dialysatseite. Austretendes Blut wird von einem (i. d. R. optischen) Sensor auf der Dialysatseite erkannt. Als Folge wird eine Alarmmeldung (optisch als Meldung auf einem Display und gegebenenfalls auch akustisch) ausgegeben. Das betreuende medizinische Personal kann eine solche Alarmmeldung durch eine Bedienereingabe für eine vorbestimmte Zeitdauer, beispielsweise 2 Minuten, aussetzen, in der die Behandlung fortgesetzt wird. Dies beruht auf der Erfahrung, dass sich kleinere Rupturen der Dialysemembran selbstständig wieder verschließen können und die Behandlung gefahrlos fortgesetzt werden kann.

Liegt das Blutleck nach dieser Aussetzzeitdauer jedoch immer noch vor (zu erkennen wiederum durch das entsprechende Sensorsignal am Blutleckdetektor), kann das Bedienpersonal aufgefordert werden, die Behandlung zu unterbrechen und den Blut- oder Dialysefilter auszutauschen.

Beim Austauschen des Dialysefilters muss der gebrauchte Dialysator entleert werden. Dabei werden die Dialysierflüssigkeitszulaufleitung und die Dialysatablaufleitung vorübergehend mit einer Kurzschlussleitung oder einem Kurzschlussstück der Blutbehandlungsvorrichtung verbunden. Die vorübergehend lose Dialysierflüssigkeitszulaufleitung und die vorübergehend ebenfalls lose Dialysatablaufleitung werden sozusagen an jeweils einem Verbinder der Kurzschlussleitung geparkt. Damit können zum einen ihre Verbinder nicht durch die Umgebung verschmutzen und zum anderen hat das Personal die Hände frei zum Austausch des Dialysefilters.

Beim bisher praktizierten Austauschen des Dialysators kann es zu einer Kontamination der Verbinder der Dialysierflüssigkeitszulaufleitung und/oder der Dialysatablaufleitung, welches meist als Hansenkupplungen ausgestaltet sind, und der Anschlüsse des Kurzschlussteils bzw. der Kurzschlussleitung mit Patientenblut kommen. Dies ist dann der Fall, wenn bei einem Blutleck Blut aus dem extrakorporalen Blutkreislauf auf die Dialysatseite übergetreten ist und damit auch an die Verbinder der Kurzschlussleitung gelangt sind.

Durch das zwischenzeitliche Umstecken der Verbinder der Dialysierflüssigkeitszulaufleitung und/oder der Dialysatablaufleitung auf die Verbinder der Kurzschlussleitung kann auch diese, inklusive ihrer Verbinder oder Anschlussstücke, kontaminiert werden. Diese Kontamination kann sowohl das Innere der Leitungen und Verbinder betreffen. Sie kann ferner die innere Oberfläche der Verbinder und nach Verbinden der Verbinder der Dialysierflüssigkeitszulaufleitung und/oder der Dialysatablaufleitung mit jenen der Kurzschlussleitung folglich auch die Kontaktfläche letzterer betreffen.

Eine derartige Kontamination spielt eine Rolle bei der späteren Behandlung eines weiteren Patienten mittels derselben Blutbehandlungsvorrichtung. Denn erfolgt eine überaus gründliche Reinigung jenseits der üblichen Reinigungsprogramme nicht, können die Dialysierflüssigkeitszulaufleitung und in Folge dessen das Dialysatkompartment des Dialysefilters mit Erregern und anderen Stoffen aus dem Blut des zum Zeitpunkt der Ruptur behandelten Patienten kontaminiert werden. Sind die fraglichen Erreger oder Stoffe membrangängig, so können sie über die Membran hinweg auch in das Blut künftig behandelter Patienten gelangen. Dies gilt es möglichst zu vermeiden.

Aus der US 5 589070 A sind ein Verfahren sowie eine Vorrichtung für die manuelle Säuberung eines Dialysekreislaufs bekannt.

Aus der US 4 153554 A ist eine Vorrichtung für den Gebrauch in einem künstlichen Nierensystem bekannt.

Aus der US 2011/089112 A1 sind ein Verfahren und eine Vorrichtung für die Entfernung von Proteinsubstanzen bekannt.

Aus der US 4897184 A ist eine Vorrichtung zur Kontrolle und Überwachung von Fluidfluss bekannt.

Eine Aufgabe der vorliegenden Erfindung ist es, eine Blutbehandlungsvorrichtung vorzuschlagen, welche das Kontaminationsrisiko im Fall eines Blutübertritts auf die Dialysatseite verringert. Zudem sollen geeignete Verfahren zum Verringern des Kontaminationsrisikos angegeben werden.

Die erfindungsgemäße Aufgabe kann durch eine Blutbehandlungsvorrichtung mit den Merkmalen des Anspruchs 1 gelöst werden. Sie kann ferner mittels der erfindungsgemäßen Verfahren mit den Merkmalen des Anspruchs 10 und des Anspruchs 12 gelöst werden.

Erfindungsgemäß wird somit eine Blutbehandlungsvorrichtung vorgeschlagen, welche optional einen Dialysefilter und/oder eine Aufnahme für einen Dialysefilter aufweist. Sie weist ferner eine Dialysierflüssigkeitszulaufleitung mit einem vorzugsweise endseitig angeordneten ersten Verbinder zum Zuführen von Dialysierflüssigkeit zum Dialysefilter und zum Verbinden mit diesem auf. Ebenso weist sie eine Dialysatablaufleitung mit einem vorzugsweise endseitig angeordneten zweiten Verbinder zum Abführen von Dialysat vom Dialysefilter und zum Verbinden mit diesem auf.

Die Blutbehandlungsvorrichtung weist des Weiteren einen Blutlecksensor auf, welcher konfiguriert und angeordnet ist zum Erkennen von Blut in oder am Dialysefilter, in der Dialysierflüssigkeitszulaufleitung und/oder in der Dialysatablaufleitung. Er ist ferner konfiguriert zum Abgeben eines Blutlecksignals, falls Blut erkannt wird.

Die Blutbehandlungsvorrichtung weist eine Kurzschlussleitung zum Verbinden der Dialysierflüssigkeitszulaufleitung und der Dialysatablaufleitung (oder mit der Dialysatablaufleitung, also direkt oder mittels Zwischenelement) in Fluidkommunikation auf. Die Kurzschlussleitung weist hierzu einen dritten Verbinder und einen vierten Verbinder, jeweils konfiguriert zum Verbinden mit jeweils einem der ersten und zweiten Verbinder, auf.

Des Weiteren weist die Blutbehandlungsvorrichtung eine Steuerung auf, welche alternativ als Regelung ausgestaltet sein kann, und welche konfiguriert ist, um ein Signal abzugeben, um im Zusammenwirken mit der Blutbehandlungsvorrichtung wenigstens ein Ausgeben einer Warnung oder eines Hinweises an den Benutzer, dass eine Reinigung des dritten und/oder vierten Verbinders erforderlich ist, ein Einleiten einer vorgeschriebene Reinigung des dritten und/oder vierten Verbinders, ein Unterbinden der Bedienbarkeit der Blutbehandlungsvorrichtung, bis der Benutzer eine oder mehrere vorgeschriebene Tätigkeiten durchgeführt hat, und ein mechanischen Verhindern eines Verbindens des dritten und/oder vierten Verbinders mit dem ersten und/oder zweiten Verbinder auszuführen, wenn ein Blutlecksignal abgegeben und/oder empfangen und/oder verarbeitet wurde. Wo im Folgenden von "abgegeben" die Rede ist, kann in gewissen erfindungsgemäßen Ausführungsformen optional auch ein "empfangen" oder "verarbeiten" zu verstehen sein.

Das erfindungsgemäße Verfahren zum Betreiben einer Blutbehandlungsvorrichtung umfasst das Bereitstellen einer Blutbehandlungsvorrichtung, welche einen Dialysefilter oder eine Aufnahme für einen Dialysefilter, eine Dialysierflüssigkeitszulaufleitung mit einem ersten Verbinder zum Zuführen von Dialysierflüssigkeit zum Dialysefilter sowie eine Dialysatablaufleitung mit einem zweiten Verbinder zum Abführen von Dialysat vom Dialysefilter aufweist. Die Blutbehandlungsvorrichtung weist ferner einen Blutlecksensor auf, welcher zum Erkennen von Blut in oder am Dialysefilter, in der Dialysierflüssigkeitszulaufleitung und/oder in der Dialysatablaufleitung, und zum Abgeben eines Blutlecksignals, falls Blut erkannt wird, dient. Des Weiteren weist sie eine Kurzschlussleitung zum Verbinden der Dialysierflüssigkeitszulaufleitung und - oder mit - der Dialysatablaufleitung in Fluidkommunikation, wobei die Kurzschlussleitung hierzu einen dritten Verbinder und einen vierten Verbinder zum Verbinden mit jeweils einem der ersten und zweiten Verbinder aufweist, und eine Steuerung auf.

Das Verfahren umfasst ein Detektieren mittels des Blutlecksensors, ob ein Blutleck vorliegt, und ein Ausgeben eines Blutlecksignals, falls dies der Fall ist. Optional kann das Blutlecksignals in einer Speichervorrichtung gespeichert werden. Das Verfahren umfasst ferner ein Überprüfen, ob ein Blutlecksignal abgegeben und/oder empfangen und/oder verarbeitet wurde und ein Abgeben eines Signals durch die Steuerung, welches im Zusammenwirken mit der Blutbehandlungsvorrichtung einen oder wenigstens einen Schritt ausführt, falls ein Blutlecksignal abgegeben und/oder empfangen und/oder verarbeitet wurde, aus der Gruppe, welche ein Ausgeben einer Warnung an den Benutzer, dass eine Reinigung des dritten und/oder vierten Verbinders erforderlich ist, ein Einleiten einer vorgeschriebenen Reinigung des dritten und/oder vierten Verbinders, ein Unterbinden der Bedienbarkeit der Blutbehandlungsvorrichtung, bis der Benutzer wenigstens eine vorgeschriebene Tätigkeit durchgeführt hat und ein mechanisches Verhindern eines Verbindens des dritten und/oder vierten Verbinders mit dem ersten und/oder zweiten Verbinder umfasst.

Das erfindungsgemäße nicht-therapeutische Verfahren zum Wechseln eines Dialysefilters einer Blutbehandlungsvorrichtung umfasst ein Bereitstellen einer Blutbehandlungsvorrichtung, welche einen ersten Dialysefilter in einer Aufnahme für einen Dialysefilter, eine Dialysierflüssigkeitszulaufleitung mit einem ersten Verbinder zum Zuführen von Dialysierflüssigkeit zum ersten Dialysefilter sowie eine Dialysatablaufleitung mit einem zweiten Verbinder zum Abführen von Dialysat vom ersten Dialysefilter aufweist. Diese weist ferner einen Blutlecksensor auf, welcher zum Erkennen von Blut in oder am ersten Dialysefilter, in der Dialysierflüssigkeitszulaufleitung und/oder in der Dialysatablaufleitung, und zum Abgeben eines Blutlecksignals falls Blut erkannt wird, dient. Sie weist ferner eine Kurzschlussleitung zum Verbinden der Dialysierflüssigkeitszulaufleitung und - oder mit - der Dialysatablaufleitung in Fluidkommunikation auf, wobei die Kurzschlussleitung hierzu einen dritten Verbinder und einen vierten Verbinder zum Verbinden mit jeweils einem der ersten und zweiten Verbinder aufweist.

Das Verfahren umfasst ferner ein Bereitstellen eines zweiten Dialysefilters, ein Trennen der Dialysierflüssigkeitszulaufleitung und der Dialysatablaufleitung vom ersten Dialysefilter und ein Verbinden der Dialysierflüssigkeitszulaufleitung und der Dialysatablaufleitung mit dem zweiten Dialysefilter, ohne die Dialysierflüssigkeitszulaufleitung oder die Dialysatablaufleitung zuvor mit der Kurzschlussleitung verbunden zu haben.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine beispielhafte erfindungsgemäße Ausführungsform erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

Erfindungsgemäße beispielhaften Ausführungsformen können eines oder mehrere der im Folgenden genannten Merkmale in beliebiger Kombination aufweisen.

In gewissen erfindungsgemäßen, beispielhaften Ausführungsformen heißt "initiieren" "veranlassen" oder "ein hierzu erforderliches Signal abgeben, welches den gewünschten Schritt im Zusammenwirken mit der Blutbehandlungsvorrichtung einleitet oder ausführt".

In bestimmten erfindungsgemäßen, beispielhaften Ausführungsformen zählt zum Erkennen von Blut auch das Erkennen Blutbestandteilen, und/oder das Erkennen einer Veränderung des Dialysats, welche einen Rückschluss auf ein vorliegendes Blutleck erlaubt. Die Veränderung kann beispielsweise eine mittels optischer Sensoren messbare Eigenschaft sein.

In manchen erfindungsgemäßen, beispielhaften Ausführungsformen weist die Blutbehandlungsvorrichtung eine Überwachungsvorrichtung auf, welche konfiguriert ist zum Erkennen eines Verbindens des dritten und/oder des vierten Verbinders, beispielsweise mit dem ersten und/oder zweiten Verbinder. Sie ist in diesen Ausführungsformen ferner konfiguriert zum Abgeben eines Verbindungssignals, falls ein Verbinden erkannt wird. Die Steuerung ist dabei konfiguriert, um wenigstens einen Schritt zu initiieren aus der o. g. Gruppe, wenn, z. B. innerhalb eines vorbestimmten Zeitraums, sowohl ein Blutlecksignal als auch ein Verbindungssignal abgegeben und/oder empfangen und/oder verarbeitet wurden.

In einigen erfindungsgemäßen, beispielhaften Ausführungsformen ist die Steuerung konfiguriert, um wenigstens einen Schritt zu initiieren aus der o. g. Gruppe, wenn, und nur dann, sowohl ein Blutlecksignal als auch ein Verbindungssignal abgegeben und/oder empfangen und/oder verarbeitet wurde.

In gewissen erfindungsgemäßen, beispielhaften Ausführungsformen der Blutbehandlungsvorrichtung weist die Überwachungsvorrichtung einen Verbindungssensor auf oder besteht hieraus. Der Verbindungssensor ist konfiguriert und angeordnet zum Erkennen eines stattgefundenen oder erfolgten Verbindens eines Verbindens mit dem dritten und/oder mit dem vierten Verbinder. Er ist zudem konfiguriert zum Abgeben eines Verbindungssignals falls ein Verbinden erkannt wird.

In manchen erfindungsgemäßen, beispielhaften Ausführungsformen der Blutbehandlungsvorrichtung ist die Überwachungsvorrichtung eine Abfragevorrichtung oder weist diese auf, mittels welcher der Benutzer auf eine Befragung, z. B. mittels Display oder Touchscreen, hin ein erfolgtes Verbinden bestätigen oder verneinen muss. Alternativ kann die Blutbehandlungsvorrichtung die Abfragevorrichtung aufweisen.

In einigen erfindungsgemäßen, beispielhaften Ausführungsformen kann, wenn der Benutzer bestätigt, dass es ein solches Verbinden gegeben hat, die Behandlung eines Patienten mittels Blutbehandlungsvorrichtung nicht fortgesetzt oder nicht wieder aufgenommen werden. Alternativ oder ergänzend kann eine Warnung ausgegeben werden, die Behandlung ausgesetzt zu lassen oder nicht mit der Behandlung eines weiteren oder nächsten Patienten zu beginnen. Die Blutbehandlungsvorrichtung und/oder Einrichtungen, insbesondere der Blutbehandlungsvorrichtung, können entsprechend eingerichtet, programmiert oder konfiguriert sein. Letztgenannte Begriffe werden in bestimmten erfindungsgemäßen Ausführungsformen als Synonyme verstanden.

In gewissen erfindungsgemäßen, beispielhaften Ausführungsformen ist die vorgeschriebene Tätigkeit aus einer Gruppe ausgewählt, welche aus einem Bestätigen, z. B. mittels Touch-Display und einem Ausführen von Reinigungsschritten durch den Benutzer, vorzugsweise mit nachfolgender Bestätigung durch diesen, dass die Reinigung erfolgt ist, besteht oder beliebige Kombination hiervon oder dergleichen umfasst.

In einigen erfindungsgemäßen, beispielhaften Ausführungsformen ist die vorgeschriebene Tätigkeit eine vorbestimmte Tätigkeit. Ihre Definition, Beschreibung oder dergleichen kann z. B. auf übliche Weise in geeigneten Einrichtungen, welche z. B. Teil der Blutbehandlungsvorrichtung oder mit dieser verbunden sein können, hinterlegt oder gespeichert sein.

In gewissen erfindungsgemäßen, beispielhaften Ausführungsformen ist die vorgeschriebene Tätigkeit nicht ein Abschalten eines Alarms, insbesondere nicht ein alleiniges Abschalten.

In manchen erfindungsgemäßen, beispielhaften Ausführungsformen weist die Blutbehandlungsvorrichtung ferner eine Verriegelung oder Abdeckung auf, welche in einem Verriegelungs- oder Abdeckzustand hiervon wenigstens den dritten und/oder den vierten Verbinder in einem Abdeckzustand derart abdeckt, dass ein Verbinden des wenigstens einen abgedeckten Verbinders mit den ersten oder dem zweiten Verbinder nicht möglich ist. Die Verriegelung ist - vorzugsweise manuell - in einen Verbindungszustand hiervon überführbar, in welchem ein Verbinden möglich ist. Dabei ist das mechanische Verhindern eines Verbindens des dritten und/oder vierten Verbinders mit dem ersten und/oder zweiten Verbinder ein Überführen in den oder ein Halten im Verriegelungs- oder Abdeckzustand. Dabei kann die Verriegelung oder Abdeckung gegen ein unzulässiges Öffnen oder Überführen in den Verbindungszustand geschützt sein, beispielsweise indem die hierzu erforderlichen Kräfte ausreichend groß bestimmt sind. Eine Option ist es, ein Öffnen oder Überführen nur unter Zerstörung von Bauteilen oder nur unter Eingeben eines Berechtigungscodes, welcher beispielsweise nur einem begrenzten Personenkreis bekannt gegeben wurde, zuzulassen.

In einigen erfindungsgemäßen, beispielhaften Ausführungsformen der Blutbehandlungsvorrichtung ist die Abdeckung eine Klappe.

In gewissen erfindungsgemäßen beispielhaften Ausführungsformen weist die Blutbehandlungsvorrichtung eine Speichervorrichtung auf, welche konfiguriert oder ansteuerbar ist, das Blutlecksignal, das nur im Falle eines Blutlecks abgegeben und/oder empfangen und/oder verarbeitet wird, über eine vorbestimmte Zeitdauer, beispielsweise bis zum Ende der Blutbehandlungssitzung, oder bis zu seiner Löschung durch den Benutzer, zu speichern. Die vorbestimmte Zeitdauer kann bis zum Eintreten eines vorbestimmten Ereignisses, insbesondere dem Beginn einer Behandlung des nächsten Patienten, andauern und mit Eintreten des vorbestimmten Ereignisses beendet sein.

In manchen erfindungsgemäßen, beispielhaften Ausführungsformen des Verfahrens weist die Blutbehandlungsvorrichtung eine Überwachungsvorrichtung zum Erkennen eines erfolgten Verbindens des dritten und/oder des vierten Verbinders, beispielsweise mit dem ersten und/oder zweiten Verbinder, und zum Abgeben eines Verbindungssignals auf. Dabei weist das Verfahren ein Detektieren mittels der Überwachungsvorrichtung auf, ob nach Ausgabe des Blutlecksignals der dritte und/oder der vierte Verbinder verbunden wurde. Es wird ein Verbindungssignal ausgegeben, falls dies der Fall ist. Dabei ist die Steuerung konfiguriert, um dann, wenn sowohl ein Blutlecksignal als auch ein Verbindungssignal abgegeben und/oder empfangen und/oder verarbeitet wurden, und nur dann, wenigstens einen Schritt zu initiieren aus der o. g. Gruppe.

In einigen erfindungsgemäßen, beispielhaften Ausführungsformen sind beide Verbinder der Kurzschlussleitung dann, wenn sie nicht verbunden sind, stets durch eine Klappe (Kurzschlussteil-Klappe) verdeckt. Ein Öffnen der Klappe wird von einem Sensor erkannt. Auch dies ist eine mögliche Form der Überwachung, ob der dritte oder vierte Verbinder möglicherweise verbunden wurde. Hier wird vereinfachend davon ausgegangen, dass ein Verbinden angesichts der nachweislich zumindest zwischenzeitlich geöffneten Klappe zumindest theoretisch möglich gewesen ist.

In gewissen erfindungsgemäßen, beispielhaften Ausführungsformen ist eine mögliche Form der Überwachung das Überprüfen, ob nach einem erfolgten Blutlecksignal ein Fluidstrom in der Kurzschlussleitung gemessen werden konnte. Hat es einen solchen Strom gegeben, so ist vereinfachend davon auszugehen, dass der dritte und der vierte Verbinder mit Verbindern der Dialysierflüssigkeitszulaufleitung und der Dialysatablaufleitung verbunden gewesen sein wird.

Erfindungsgemäße Ausführungsformen können einen oder mehrere der hierin genannten Vorteile aufweisen. Zu diesen zählen insbesondere die im Folgenden genannten.

Erfindungsgemäß kann die Sicherheit eines nachfolgenden Patienten erhöht werden, da ein Verbinden von kontaminierten Verbindern mit der Kurzschlussleitung erkannt wird und Gegenmaßnahmen ergriffen werden können. In manchen erfindungsgemäßen Ausführungsformen kann die erzielte Sicherheit sogar noch erhöht werden, indem die vorliegende Erfindung vorsieht, dass Gegenmaßnahmen zur Vermeidung oder Beseitigung der Kontamination unabdingbar ergriffen werden müssen, da ansonsten die Blutbehandlungsvorrichtung bis auf weiteres nicht für weitere Patienten verwendbar ist.

Steckt der Benutzer bei Auftreten eines Blutlecks mit anschließendem Wechsel des Dialysefilters oder Dialysators die Dialysierflüssigkeitszulaufleitung und die Dialysatablaufleitung wie erfindungsgemäß vorgeschlagen nicht mehr auf die Verbinder der Kurzschlussleitung sondern direkt vom alten, ersten Dialysefilter zum neuen Dialysefilter, so wird zuverlässig verhindert, dass die Kurzschlussleitung oder andere Komponenten der Blutbehandlungsvorrichtung kontaminiert werden.

Doch selbst für den Fall, dass sich der Benutzer nicht an dieses für den Austausch des Dialysefilters mit dem Blutleck erfindungsgemäß empfohlene Vorgehen gehalten haben sollte, besteht Kontaminationsschutz da sich die erfindungsgemäße Blutbehandlungsvorrichtung (etwa durch Setzen eines entsprechenden *flag*) merkt, dass nach einem Blutleckalarm die Dialysierflüssigkeitszulaufleitung und die Dialysatablaufleitung auf die Kurzschlussleitung gesteckt waren, beispielsweise wie oben diskutiert indem entsprechende Sensoren eine Verbindung detektieren, oder aber auch durch eine entsprechende Eingabe, die der Bediener nach dem Wechsel des Dialysefilters in die Bedienerschnittstelle des Dialysegeräts eingibt (beispielsweise durch Ausgabe einer entsprechenden Frage auf einer Abfragevorrichtung wie beispielsweise dem Display: "Wurden die Dialysierflüssigkeitszulaufleitung und/oder die Dialysatablaufleitung auf das Kurzschlussteil gesteckt?" Auswahlmöglichkeit beispielsweise per Touchbutton "ja", "nein").

Abhängig von der vom Bediener durchgeführten Handlung, also abhängig davon, ob der Bediener nach einem Blutleckalarm die Dialysierflüssigkeitszulaufleitung und/oder die Dialysatablaufleitung auf die Verbinder der Kurzschlussleitung gesteckt hat, wird vor der nächsten Behandlung eine entsprechende Reinigungsprozedur verlangt, um sicher zu stellen, dass alle möglichen Kontaminationsstellen desinfiziert sind. Diese können ein Eintauchen der Verbinder in Desinfektionslösung, Oberflächendesinfektion der Verbinder der Kurzschlussleitung und mehr umfassen.

Die erfindungsgemäße Blutbehandlungsvorrichtung kann bei entsprechend konfigurierter Steuerung diese Schritte durch die Ausgabe von Fragen an den Bediener und erwartete Eingaben, ohne die keine Behandlung möglich ist, protokollieren. So wird das Bedienpersonal vorteilhaft auf die besondere Situation (Blutleck in der vorhergehenden Behandlung) aufmerksam gemacht und sensibilisiert.

Die vorliegende Erfindung wird im Folgenden mit Bezug auf die in den angehängten Figuren gezeigten, rein exemplarischen Ausführungsbeispiele beschrieben, welche nicht als beschränkend verstanden werden sollen. In den Figuren bezeichnen identische Bezugszeichen gleiche oder identische Elemente. Es gilt:
- **Fig. 1**: zeigt schematisch sehr vereinfacht Abschnitte einer erfindungsgemäßen Blutbehandlungsvorrichtung in einer ersten Ausführungsform;
- **Fig. 2**: zeigt schematisch sehr vereinfacht Abschnitte einer erfindungsgemäßen Blutbehandlungsvorrichtung in einer zweiten Ausführungsform und im Verbindungszustand; und
- **Fig. 3**: zeigt die Abschnitte der erfindungsgemäßen Blutbehandlungsvorrichtung aus Fig. 2 im Abdeckungs- oder Verriegelungszustand; und
- **Fig. 4**: zeigt das erfindungsgemäße Verfahren in einer beispielhaften Ausführungsform.

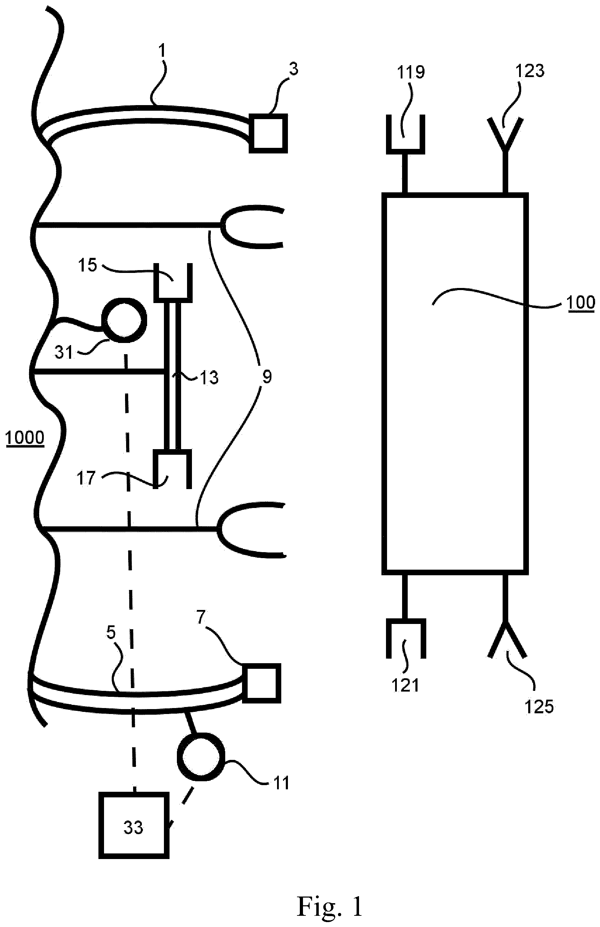

**Fig. 1** zeigt schematisch sehr vereinfacht Abschnitte einer erfindungsgemäßen Blutbehandlungsvorrichtung 1000 in einer ersten Ausführungsform.

Die Blutbehandlungsvorrichtung 1000 weist eine Dialysierflüssigkeitszulaufleitung 1 mit einem ersten Verbinder 3 und eine zweite Dialysatablaufleitung 5 mit einem zweiten Verbinder 7 auf. Sowohl der erste als auch der zweite Verbinder 3, 7 können optional als Hansenkupplungen oder als andere Verbindertypen ausgestaltet sein.

Die Blutbehandlungsvorrichtung 1000 weist ferner eine, hier optional mehrteilige, Aufnahme 9 zum lösbaren Aufnehmen eines Dialysefilters 100 mit einer Blutseite und einer Dialysatseite auf. Die Dialysatablaufleitung 5 weist einen Blutlecksensor 11 zum Erkennen von Blut im Inneren der Dialysatablaufleitung 5 auf. Der Blutlecksensor 11 kann jedoch alternativ oder ergänzend an jeder anderen Stelle vorgesehen sein, an welcher der Übertritt von Blut aus der Blutseite des Dialysefilters 100 auf dessen Dialysatseite erkannt werden könnte.

Des Weiteren weist die Blutbehandlungsvorrichtung 1000 eine Kurzschlussleitung 13 mit einem dritten Verbinder 15 und einem vierten Verbinder 17 auf. Sowohl der dritte als auch der vierte Verbinder 13, 17 können optional als Teil oder Gegenstück zu Hansenkupplungen oder als andere Verbindertypen ausgestaltet sein.

Der Dialysefilter 100 weist ebenfalls Verbinder 119, 121 auf, welche ausgestaltet sind, um mit dem ersten und zweiten Verbinder 3, 7 verbunden zu werden. Des Weiteren weist der Dialysefilter 100 hier nicht weiter relevante Verbinder 123, 125 zur Verbindung mit der arteriellen und der venösen Leitung auf.

Der erste und der zweite Verbinder 3, 7 können zur Behandlung des Patienten mit den Verbindern 119, 121 der Dialysatseite des Dialysefilters 100 mit letzterem verbunden werden, wie dies bei und zu der Patientenbehandlung üblich und erforderlich ist.

In bestimmten Situationen wie dem Austausch des Dialysefilters 100 sollen der erste und der zweite Verbinder 3, 7 vom Dialysefilter 100 gelöst werden. Der Dialysefilter 100 ist somit von der Blutbehandlungsvorrichtung 1000 abgekoppelt. Der erste und der zweite Verbinder 3, 7 werden anschließend mit dem dritten Verbinder 15 und dem vierten Verbinder 17 der Kurzschlussleitung 13 in Fluidkommunikation verbunden. In dieser Stellung könnten die Leitungen 1 und 5 auf Wunsch sogar bei abgekoppeltem Dialysefilter 100 gespült werden.

Für den Fall, dass mittels des Blutlecksensors 11 jedoch Blut auf der Dialysatseite, hier vornehmlich in der Dialysatablaufleitung 5, erkannt wird, sind die ersten und zweiten Verbinder 3 und 7 zwar ebenfalls vom Dialysefilter 100 zu lösen, damit dieser ausgetauscht werden kann. Während letzterer ausgetauscht wird, sollen die ersten und zweiten Verbinder 3 und 7 im Falle des Blutalarms gerade nicht auf den dritten Verbinder 15 und den vierten Verbinder 17 der Kurzschlussleitung 13 aufgesetzt werden, und die Leitungen 1 und 5 dürfen nicht mittels Kurzschlussleitung 13 gespült werden.

Um sicherzustellen, dass die Leitungen 1 und 5 bei Erkennen von Blut durch den Blutlecksensor 11 nicht mittels Kurzschlussleitung 13 gespült bzw. mit dieser verbunden werden, weist die erfindungsgemäße Blutbehandlungsvorrichtung 1000 eine Überwachungsvorrichtung 31 auf. Die Überwachungsvorrichtung 31 der Fig. 1 ist als ein Verbindungssensor ausgestaltet oder weist diesen auf, welcher eine erfolgtes Verbinden der Leitungen 1 und 5 mit der Kurzschlussleitung 13 erkennt und mittels Verbindungssignal entsprechend an eine Steuerung 33 meldet. Da die Steuerung 33 in der in Fig. 1 gezeigten Ausführungsform auch mit dem Blutlecksensor 11 verbunden ist, was in den Figuren durch Strichlinien angedeutet ist, treffen bei ihr die Information, dass es ein Blutleck gegeben hat, und die Information, dass hieran anschließend die Leitungen 1, 5 mit der Kurzschlussleitung 13 verbunden wurden, zusammen. Die Steuerung 33 kann in Folge dessen im Zusammenwirken mit den hierzu jeweils erforderlichen Einrichtungen beispielsweise eine Warnung an den Benutzer ausgeben, eine Reinigung des dritten und/oder vierten Verbinders 15, 17 einleiten, die Blutbehandlungsvorrichtung 1000 bis auf weiteres für Patientenbehandlungen blockieren, oder andere Maßnahmen einleiten oder warnende Hinweise abgeben.

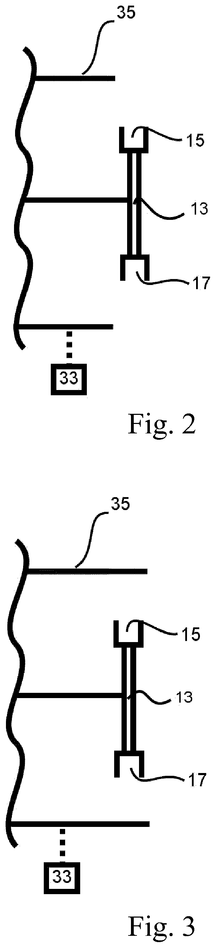

**Fig. 2** zeigt schematisch sehr vereinfacht Abschnitte einer erfindungsgemäßen Blutbehandlungsvorrichtung in einer zweiten Ausführungsform. Zu erkennen ist eine Verriegelung 35, welche angeordnet ist, in ihrem Abdeckungs- oder Verriegelungszustand, welcher in Fig. 3 gezeigt ist, ein Verbinden von Verbindern mit dem dritten und/oder dem vierten Verbinder 15, 17 der Kurzschlussleitung 13 mechanisch zu verhindern. In Fig. 2 ist die Verriegelung 35 allerdings in ihrem Verbindungszustand gezeigt, in welchem ein solches Verbinden nicht unterbunden ist.

**Fig. 3** zeigt den Gegenstand der Fig. 2. Zu erkennen ist, dass die Verriegelung 35, welche in ihrem Verriegelungszustand gezeigt ist, ein Verbinden von Verbindern mit dem dritten und/oder dem vierten Verbinder 15, 17 der Kurzschlussleitung 13 wie gewünscht mechanisch verhindert. In Fig. 3 geschieht letzteres durch ein Verlegen oder Blockieren des Zugangs zu den Verbindern 15, 17. Dabei ist jedes andere mechanische Verhindern eines Verbindens ebenfalls von der vorliegenden Erfindung umfasst.

Zu einem solchen mechanischen Verhindern zählt auch der Einsatz einer hier nicht gezeigten Klappe, welche abdeckend über die Verbinder 15, 17 und damit in einen Verriegelungszustand klappbar ist.

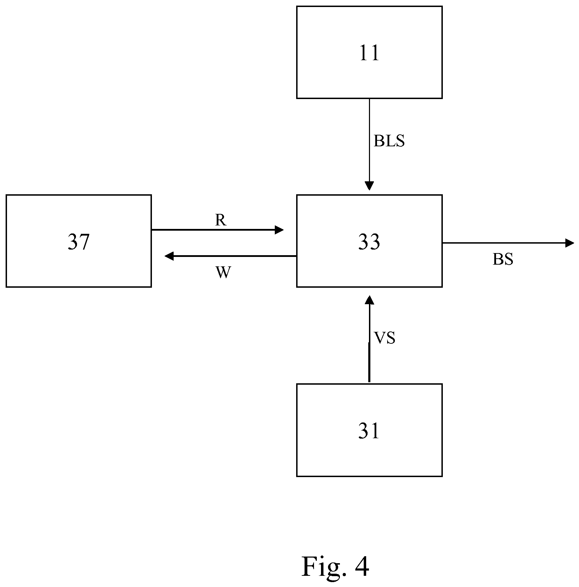

**Fig. 4** zeigt das erfindungsgemäße Verfahren in einer beispielhaften Ausführungsform.

In einem ersten Schritt erkennt der Blutlecksensor 11 den Übertritt von Blut aus der Blutseite des Dialysefilters 100 in dessen Dialysatseite, was zur Abgabe eines Blutlecksignals BLS an die Steuerung 33 führt.

Die Steuerung 33 oder eine hiermit verbundene Speichervorrichtung 37 kann das Blutlecksignal BLS auf Wunsch über eine vorbestimmte Zeitdauer, bis zur Beendigung der Blutbehandlungssitzung, speichern, beispielsweise über das Setzen eines sog. *tag.* Die Steuerung 33 steht in dem Fall, dass eine Speichervorrichtung 37 vorgesehen ist, mit dieser mittels Signale R und W in Lese- und Schreibeverbindung.

Falls die Überwachungsvorrichtung 31 in einem späteren Schritt das Verbinden des dritten und/oder vierten Verbinders 15, 17 mit weiteren Verbindern, beispielsweise dem ersten oder zweiten Verbinder 3, 7 erkennt, so gibt sie ein Verbindungssignal VS an die Steuervorrichtung 33 ab, welches ein erfolgtes Verbinden dokumentiert.

Die Steuervorrichtung 33 prüft kontinuierlich oder in vorgegebenen Zeitabständen, ob sie sowohl ein Blutlecksignal BLS als auch ein Verbindungssignal VS erhalten und ggf. gespeichert hat. Ist das Ergebnis der Prüfung positiv, d. h. liegen beide Signale vor, so ist von einer Gefahr der Kontaminierung des dritten und/oder vierten Verbinders 15, 17 auszugehen. In diesem Fall leitet die Steuervorrichtung 33 mittels eines Betätigungssignals BS eine oder mehrere der hierin beschriebenen Schritte zur Gefahrenabwendung ein. Zu diesen zählt das Ausgeben einer Warnung, das Einleiten einer Reinigung, ein Blockieren der Benutzung der Blutbehandlungsvorrichtung 1000 bis auf weiteres, oder ein mechanisches Verriegeln oder Abdecken des dritten und/oder vierten Verbinders 15, 17. Die Steuervorrichtung 33 ist hierzu mit den erforderlichen Vorrichtungen der Blutbehandlungsvorrichtung 1000 verbunden oder steht mit diesen in Signalverbindung.

### Bezugszeichen

- 1000: Blutbehandlungsvorrichtung
- 100: Dialysefilter
- 1: Dialysierflüssigkeitszulaufleitung
- 3: erster Verbinder
- 5: Dialysatablaufleitung
- 7: zweiter Verbinder
- 9: Aufnahme
- 11: Blutlecksensor
- 13: Kurzschlussleitung oder Kurzschlussstück
- 15: dritter Verbinder
- 17: vierter Verbinder
- 119, 121: Verbinder des Dialysefilters auf der Dialysatseite
- 123, 125: Verbinder des Dialysefilters auf der Blutseite
- 31: Überwachungsvorrichtung
- 33: Steuerung oder Regelung
- 35: Verriegelung oder Abdeckung
- 37: Speichervorrichtung

- BLS: Blutlecksignal
- BS: Betätigungssignal
- R: Lesesignal
- VS: Verbindungssignal
- W: Schreibsignal

## Patentansprüche

1. Blutbehandlungsvorrichtung (1000) mit
- einem Dialysefilter (100) oder einer Aufnahme (9) für einen Dialysefilter (100);
- einer Dialysierflüssigkeitszulaufleitung (1) mit einem ersten Verbinder (3) zum Zuführen von Dialysierflüssigkeit zum Dialysefilter (100);
- einer Dialysatablaufleitung (5) mit einem zweiten Verbinder (7) zum Abführen von Dialysat vom Dialysefilter (100);
- einem Blutlecksensor (11) konfiguriert und angeordnet zum Erkennen von Blut in oder am Dialysefilter (100), in der Dialysierflüssigkeitszulaufleitung (1) und/oder in der Dialysatablaufleitung (5), und konfiguriert zum Abgeben eines Blutlecksignals (BLS) falls Blut erkannt wird;
- einer Kurzschlussleitung (13) zum Verbinden der Dialysierflüssigkeitszulaufleitung (1) und, oder mit, der Dialysatablaufleitung (5) in Fluidkommunikation, wobei die Kurzschlussleitung (13) hierzu einen dritten Verbinder (15) und einen vierten Verbinder (17) zum Verbinden mit jeweils einem der ersten und zweiten Verbinder (3, 7) aufweist;
- einer Steuerung (33), welche konfiguriert ist, um das Blutlecksignal (BLS) zu empfangen und
**gekennzeichnet dadurch, dass** die Steuerung des Weiteren konfiguriert ist zum Abgeben eines Signals, welches im Zusammenwirken mit der Blutbehandlungsvorrichtung (1000) einen oder wenigstens einen Schritt ausführt aus der Gruppe, welche aus
- Ausgeben einer Warnung an den Benutzer, dass eine Reinigung des dritten und/oder vierten Verbinders (15, 17) erforderlich ist;
- Einleiten einer vorgeschriebenen oder vorbestimmten Reinigung des dritten und/oder vierten Verbinders (15, 17);
- Unterbinden der Bedienbarkeit der Blutbehandlungsvorrichtung (1000), bis der Benutzer mindestens eine vorgeschriebene Tätigkeit durchgeführt hat; und
- mechanisches Verhindern eines Verbindens des dritten und/oder vierten Verbinders (15, 17) mit dem ersten und/oder zweiten Verbinder (3, 7) ;
besteht, wenn ein Blutlecksignal (BLS) abgegeben und/oder empfangen wurde.

2. Blutbehandlungsvorrichtung (1000) nach Anspruch 1, ferner mit einer Überwachungsvorrichtung (31), welche konfiguriert ist zum Erkennen eines Verbindens des dritten und/oder des vierten Verbinders (15, 17) und welcher ferner konfiguriert ist zum Abgeben und/oder Empfangen eines Verbindungssignals (VS) falls ein Verbinden erkannt wird, wobei die Steuerung (33) konfiguriert ist, um wenigstens einen Schritt zu initiieren aus der Gruppe, wenn sowohl ein Blutlecksignal (BLS) als auch ein Verbindungssignal (VS) abgegeben und/oder empfangen wurden.

3. Blutbehandlungsvorrichtung (1000) nach Anspruch 2, wobei die Überwachungsvorrichtung einen Verbindungssensor aufweist oder hieraus besteht, welcher konfiguriert und angeordnet ist zum Erkennen eines erfolgten Verbindens mit dem dritten und/oder dem vierten Verbinder (15, 17), und wobei die Überwachungsvorrichtung konfiguriert ist zum Abgeben eines Verbindungssignals (VS) falls ein Verbinden erkannt wird.

4. Blutbehandlungsvorrichtung (1000) nach einem der vorangegangenen Ansprüche, wobei die Blutbehandlungsvorrichtung (1000) eine Abfragevorrichtung aufweist oder ist, mittels welcher der Benutzer auf eine Befragung hin ein erfolgtes Verbinden bestätigen und/oder verneinen kann oder muss, wenn ein Blutlecksignal (BLS) abgegeben und/oder empfangen wurde.

5. Blutbehandlungsvorrichtung (1000) nach Anspruch 4, wobei im Falle des Bestätigens die Behandlung eines Patienten mittels der Blutbehandlungsvorrichtung (1000) nicht fortgesetzt oder nicht wieder aufgenommen werden kann oder eine Warnung ausgegeben wird, die Behandlung ausgesetzt zu lassen oder nicht neu zu beginnen.

6. Blutbehandlungsvorrichtung (1000) nach einem der vorangegangenen Ansprüche, wobei die vorgeschriebene Tätigkeit aus einer Gruppe ausgewählt ist, welche aus einer Eingabe und einem Ausführen von Reinigungsschritten durch den Benutzer besteht.

7. Blutbehandlungsvorrichtung (1000) nach einem der vorangegangenen Ansprüche, wobei die Blutbehandlungsvorrichtung (1000) ferner eine Verriegelung oder Abdeckung (35) aufweist, welche in einem Verriegelungs- oder Abdeckzustand hiervon wenigstens einen von dem dritten und dem vierten Verbinder (15, 17) in einem Abdeckzustand derart abdeckt, dass ein Verbinden des wenigstens einen verriegelten oder abgedeckten Verbinders (15, 17) mit den ersten oder dem zweiten Verbinder (3, 7) nicht möglich ist, und welche in einen Verbindungszustand hiervon überführbar ist, in welchem ein Verbinden möglich ist, wobei das mechanische Verhindern eines Verbindens des dritten und/oder vierten Verbinders (15, 17) mit dem ersten und/oder zweiten Verbinder (3, 7) ein Überführen der Verriegelung oder der Abdeckung (35) in den Verriegelungs- oder Abdeckzustand oder Halten hierin ist.

8. Blutbehandlungsvorrichtung (1000) nach Anspruch 7, wobei die Abdeckung (35) eine Klappe ist.

9. Blutbehandlungsvorrichtung (1000) nach einem der vorangegangenen Ansprüche, welche eine Speichervorrichtung (37) aufweist, welche konfiguriert oder angesteuert ist, das Blutlecksignal (BLS) über eine vorbestimmte Zeitdauer oder bis zum Eintreten eines vorbestimmten Ereignisses zu speichern.

10. Verfahren zum Betreiben einer Blutbehandlungsvorrichtung (1000), mit den Schritten:
- Bereitstellen einer Blutbehandlungsvorrichtung (1000), welche aufweist:
- einen Dialysefilter (100) oder eine Aufnahme (9) für einen Dialysefilter (100);
- eine Dialysierflüssigkeitszulaufleitung (1) mit einem ersten Verbinder (3) zum Zuführen von Dialysierflüssigkeit zum Dialysefilter (100);
- eine Dialysatablaufleitung (5) mit einem zweiten Verbinder (7) zum Abführen von Dialysat vom Dialysefilter (100);
- einen Blutlecksensor (11) zum Erkennen von Blut in oder am Dialysefilter (100), in der Dialysierflüssigkeitszulaufleitung (1) und/oder in der Dialysatablaufleitung (5), und zum Abgeben eines Blutlecksignals falls Blut erkannt wird;
- eine Kurzschlussleitung (13) zum Verbinden der Dialysierflüssigkeitszulaufleitung (1) und - oder mit - der Dialysatablaufleitung (5) in Fluidkommunikation, wobei die Kurzschlussleitung (13) hierzu einen dritten Verbinder (15) und einen vierten Verbinder (17) zum Verbinden mit jeweils einem der ersten und zweiten Verbinder (3, 7) aufweist;
- eine Steuerung (33);
wobei das Verfahren weiter folgende Schritte umfasst:
- Detektieren mittels des Blutlecksensors (11), ob ein Blutleck vorliegt, und Ausgeben eines Blutlecksignals (BLS) falls dies der Fall ist;
- Überprüfen, ob ein Blutlecksignal (BLS) abgegeben und/oder empfangen und/oder verarbeitet wurde;
- Abgeben eines Signals durch die Steuerung (33), welches im Zusammenwirken mit der Blutbehandlungsvorrichtung (1000) einen oder wenigstens einen Schritt ausführt, falls ein Blutlecksignal (BLS) abgegeben und/oder empfangen und/oder verarbeitet wurde, aus der Gruppe, welche folgende Schritte umfasst:
- Ausgeben einer Warnung an den Benutzer, dass eine Reinigung des dritten und/oder vierten Verbinders (15, 17) erforderlich ist;
- Einleiten einer vorbestimmten Reinigung des dritten und/oder vierten Verbinders (15, 17);
- Unterbinden der Bedienbarkeit der Blutbehandlungsvorrichtung (1000), bis der Benutzer wenigstens eine vorgeschriebene Tätigkeit durchgeführt hat; und
- mechanisches Verhindern eines Verbindens des dritten und/oder vierten Verbinders (15, 17) mit dem ersten und/oder zweiten Verbinder (3, 7) .

11. Verfahren nach Anspruch 10, wobei die Blutbehandlungsvorrichtung (1000) eine Überwachungsvorrichtung (31) zum Erkennen eines Verbindens des dritten und/oder des vierten Verbinders (15, 17) und zum Abgeben eines Verbindungssignals (VS) aufweist; und wobei das Verfahren den weiteren Schritt aufweist:
- Detektieren mittels der Überwachungsvorrichtung (31) ob nach Ausgabe des Blutlecksignals (BLS) der dritte und/oder dem vierte Verbinder (15, 17) verbunden wurde, und Ausgeben eines Verbindungssignals (VS) falls dies der Fall ist;
wobei die Steuerung (33) konfiguriert ist, um wenigstens einen Schritt zu initiieren aus der Gruppe, wenn sowohl ein Blutlecksignal (BLS) als auch ein Verbindungssignal (VS) abgegeben und/oder empfangen und/oder verarbeitet wurden.

12. Nicht-therapeutisches Verfahren zum Wechseln eines Dialysefilters (100) einer Blutbehandlungsvorrichtung (1000), mit den Schritten:
- Bereitstellen einer Blutbehandlungsvorrichtung (1000), welche aufweist:
- einen ersten Dialysefilter (100) in einer Aufnahme (9) für einen Dialysefilter (100);
- eine Dialysierflüssigkeitszulaufleitung (1) mit einem ersten Verbinder (3) zum Zuführen von Dialysierflüssigkeit zum ersten Dialysefilter (100);
- eine Dialysatablaufleitung (5) mit einem zweiten Verbinder (7) zum Abführen von Dialysat vom ersten Dialysefilter (100);
- einen Blutlecksensor (11) zum Erkennen von Blut in oder am ersten Dialysefilter (100), in der Dialysierflüssigkeitszulaufleitung (1) und/oder in der Dialysatablaufleitung (5), und zum Abgeben eines Blutlecksignals falls Blut erkannt wird;
- eine Kurzschlussleitung (13) zum Verbinden der Dialysierflüssigkeitszulaufleitung (1) und oder mit der Dialysatablaufleitung (5) in Fluidkommunikation, wobei die Kurzschlussleitung (13) hierzu einen dritten Verbinder (15) und einen vierten Verbinder (17) zum Verbinden mit jeweils einem der ersten und zweiten Verbinder (3, 7) aufweist;
- einer Steuerung (33), welche konfiguriert ist, um das Blutlecksignal (BLS) zu empfangen und Abgeben eines Signals durch die Steuerung (33), welches im Zusammenwirken mit der Blutbehandlungsvorrichtung (1000) einen oder wenigstens einen Schritt ausführt aus der Gruppe, welche aus
- Ausgeben einer Warnung an den Benutzer, dass eine Reinigung des dritten und/oder vierten Verbinders (15, 17) erforderlich ist;
- Einleiten einer vorgeschriebenen oder vorbestimmten Reinigung des dritten und/oder vierten Verbinders (15, 17);
- Unterbinden der Bedienbarkeit der Blutbehandlungsvorrichtung (1000), bis der Benutzer mindestens eine vorgeschriebene Tätigkeit durchgeführt hat; und
- mechanisches Verhindern eines Verbindens des dritten und/oder vierten Verbinders (15, 17) mit dem ersten und/oder zweiten Verbinder (3, 7) ;
besteht, wenn ein Blutlecksignal (BLS) abgegeben und/oder empfangen wurde,
wobei das Verfahren weiter folgende Schritte umfasst:
- Bereitstellen eines zweiten Dialysefilters (100);
- Trennen der Dialysierflüssigkeitszulaufleitung (1) und der Dialysatablaufleitung (5) vom ersten Dialysefilter (100);
- Verbinden der Dialysierflüssigkeitszulaufleitung (1) und der Dialysatablaufleitung (5) mit dem zweiten Dialysefilter (100), ohne die Dialysierflüssigkeitszulaufleitung (1) oder die Dialysatablaufleitung (5) zuvor mit der Kurzschlussleitung (13) verbunden zu haben.

## Claims

1. A blood treatment apparatus (1000) comprising:
- a dialysis filter (100) or a receptacle (9) for a dialysis filter (100);
- a dialysis fluid supply line (1) having a first connector (3) for supplying dialysis fluid to the dialysis filter (100);
- a dialysate discharge line (5) having a second connector (7) for discharging dialysate from the dialysis filter (100);
- a blood leak sensor (11) configured and arranged to detect blood in or on the dialysis filter (100), in the dialysis fluid supply line (1) and/or in the dialysate discharge line (5), and configured to emit a blood leak signal (BLS) if blood is detected;
- a short-circuit line (13) for connecting the dialysis fluid supply line (1) and, or with, the dialysate discharge line (5) in fluid communication,
the short-circuit line (13) comprising hereto a third connector (15) and a fourth connector (17) intended to be respectively connected with one of the first and second connectors (3, 7);
- a controller (33) configured to receive the blood leak signal (BLS) and **characterized in that** the controller is further configured to emit a signal which, in cooperation with the blood treatment apparatus (1000), performs one, or at least one step from the group consisting of:
- issuing a warning to the user that a cleaning of the third and/or fourth connector/s (15, 17) is necessary;
- initiating a required or predetermined cleaning of the third and/or fourth connector/s (15, 17);
- preventing the usability of the blood treatment apparatus (1000), until the user has performed at least one required activity, and
- mechanically preventing the connection of the third and/or fourth connector/s (15, 17) with the first and/or second connector/s (3, 7);
when a blood leak signal (BLS) has been emitted and/or received.

2. The blood treatment apparatus (1000) according to claim 1, further comprising a monitoring apparatus (31), which is configured to detect a connection of the third and/or fourth connector/s (15, 17) and which is further configured to emit and/or receive a connection signal (VS) if a connection is detected, wherein the controller (33) is configured to initiate at least one step from the group when both a blood leak signal (BLS) and a connection signal (VS) have been emitted and/or received.

3. The blood treatment apparatus (1000) according to claim 2, wherein the monitoring apparatus comprises or consists of a connection sensor configured and arranged to detect a connection established with the third and/or fourth connector/s (15, 17), and wherein the monitoring apparatus is configured to emit a connection signal (VS) if a connection is detected.

4. The blood treatment apparatus (1000) according to anyone of the preceding claims, wherein the blood treatment apparatus (1000) comprises or is a querying apparatus, by means of which, in response to a query, the user can or must confirm and/or deny a connection established, when a blood leak signal (BLS) has been emitted and/or received.

5. The blood treatment apparatus (1000) according to claim 4, wherein, in the case of confirmation, the treatment of a patient by means of the blood treatment apparatus (1000) cannot be continued or resumed, or a warning is emitted to interrupt the treatment or not to restart it.

6. The blood treatment apparatus (1000) according to anyone of the preceding claims, wherein the required action is selected from a group consisting of an input and an execution of cleaning steps by the user.

7. The blood treatment apparatus (1000) according to anyone of the preceding claims, wherein the blood treatment apparatus (1000) further comprises a latch or a cover (35) which, in a latching or covering state thereof, covers at least one of the third and fourth connector/s (15, 17) in a covering state such that a connection of at least one latched or covered connector (15, 17) with the first or second connector (3, 7) is not possible, and which is transferable into a connection state thereof, in which a connection is possible,
wherein mechanically preventing a connection of the third and/or fourth connector/s (15, 17) with the first and/or second connector/s (3, 7) consists in transferring the latch or the cover (35) into the latching or covering state or holding therein.

8. The blood treatment apparatus (1000) according to claim 7, wherein the cover (35) is a flap.

9. The blood treatment apparatus (1000) according to anyone of the preceding claims, comprising a memory means (37) configured or controlled to memorize the blood leak signal (BLS) for a predetermined period of time or until a predetermined event occurs.

10. A method of operating a blood treatment apparatus (1000), comprising the steps of:
- providing a blood treatment apparatus (1000) comprising:
- a dialysis filter (100) or a receptacle (9) for a dialysis filter (100);
- a dialysis fluid supply line (1) having a first connector (3) for supplying dialysis fluid to the dialysis filter (100);
- a dialysate discharge line (5) having a second connector (7) for discharging dialysate from the dialysis filter (100);
- a blood leak sensor (11) for detecting blood in or on the dialysis filter (100), in the dialysis fluid supply line (1) and/or in the dialysate discharge line (5) and for emitting a blood leak signal if blood is detected;
- a short-circuit line (13) for connecting the dialysis fluid supply line (1) and, or with, the dialysate discharge line (5) in fluid communication,
the short-circuit line (13) comprising hereto a third connector (15) and a fourth connector (17) intended to be respectively connected with one of the first and second connectors (3, 7);
- a controller (33)
wherein the method further comprises the steps of:
- detecting by means of the blood leak sensor (11), whether there is a blood leak and emitting a blood leak signal (BLS) if this is the case;
- verifying that a blood leak signal (BLS) has been emitted and/or received and/or processed;
- emitting a signal by means of the controller (33) which, in cooperation with the blood treatment apparatus (1000), when a blood leak signal (BLS) has been emitted and/or received and/or processed, performs one step or at least one step from the group, comprising the steps of:
- issuing a warning to the user that a cleaning of the third and/or fourth connector/s (15, 17) is necessary;
- initiating a required or predetermined cleaning of the third and/or fourth connector/s (15, 17);
- preventing the usability of the blood treatment apparatus (1000) until the user has performed at least one required activity; and
- mechanically preventing the connection of the third and/or fourth connector/s (15, 17) with the first and/or second connector/s (3, 7).

11. The method according to claim 10, wherein the blood treatment apparatus (1000) comprises a monitoring apparatus (31) for detecting a connection of the third and/or fourth connector/s (15, 17) and for emitting a connection signal (VS); and wherein the method further comprises the step of:
- detecting by means of the monitoring apparatus (31), whether the third and/or fourth connector/s (15, 17) has/have been connected after emission of the blood leak signal (BLS), and emitting a connection signal (VS) if this is the case;
wherein the controller (33) is configured to initiate at least one step from the group when both a blood leak signal (BLS) and a connection signal (VS) have been emitted and/or received and/or processed.

12. Non-therapeutic method for changing a dialysis filter (100) of a blood treatment apparatus (1000), comprising the steps of:
- providing a blood treatment apparatus (1000) comprising:
- a first dialysis filter (100) in a receptacle (9) for a dialysis filter (100);
- a dialysis fluid supply line (1) having a first connector (3) for supplying dialysis fluid to the first dialysis filter (100);
- a dialysate discharge line (5) having a second connector (7) for discharging dialysate from the first dialysis filter (100);
- a blood leak sensor (11) for detecting blood in or on the first dialysis filter (100), in the dialysis fluid supply line (1) and/or in the dialysate discharge line (5) and for emitting a blood leak signal if blood is detected;
- a short-circuit line (13) for connecting the dialysis fluid supply line (1) and, or with, the dialysate discharge line (5) in fluid communication,
the short-circuit line (13) comprising hereto a third connector (15) and a fourth connector (17) intended to be respectively connected with one of the first and second connectors (3, 7);
- a controller (33) configured to receive the blood leak signal (BLS) and to emit a signal by means of the controller (33), which, in cooperation with the blood treatment apparatus (1000), performs one step or at least one step from the group consisting of:
- issuing a warning to the user that a cleaning of the third and/or fourth connector/s (15, 17) is necessary;
- initiating a required or predetermined cleaning of the third and/or fourth connector/s (15, 17);
- preventing the usability of the blood treatment apparatus (1000) until the user has performed at least one required activity; and
- mechanically preventing the connection of the third and/or fourth connector/s (15, 17) with the first and/or second connector/s (3, 7).
when a blood leak signal (BLS) has been emitted and/or received,
wherein the method further comprises the steps of:
- providing a second dialysis filter (100);
- separating the dialysis fluid supply line (1) and the dialysate discharge line (5) from the first dialysis filter (100);
- connecting the dialysis fluid supply line (1) and the dialysate discharge line (5) with the second dialysis filter (100), without previously connecting the dialysis fluid supply line (1) or the dialysate discharge line (5) with the short-circuit line (13).

## Revendications

1. Un appareil de traitement du sang (1000) comprenant:
- un filtre de dialyse (100) ou un réceptacle (9) pour un filtre de dialyse (100);
- un conduit d'alimentation en fluide de dialyse (1) ayant un premier connecteur (3) pour alimenter le filtre de dialyse (100) en fluide de dialyse;
- un conduit d'évacuation de dialysat (5) ayant un second connecteur (7) pour évacuer le dialysat du filtre de dialyse (100);
- un capteur de fuite de sang (11) configuré et agencé pour détecter du sang dans ou sur le filtre de dialyse (100), dans le conduit d'alimentation en fluide de dialyse (1) et/ou dans le conduit d'évacuation de dialysat (5),
et configuré pour émettre un signal de fuite de sang (BLS) si du sang est détecté;
- un conduit court-circuit (13) pour relier le conduit d'alimentation en fluide de dialyse (1) et le, ou au, conduit d'évacuation de dialysat (5) en communication fluidique, le conduit court-circuit (13) comprenant pour ce faire un troisième connecteur (15) ainsi qu'un quatrième connecteur (17) destinés à être reliés respectivement à l'un du premier et du second connecteur (3, 7);
- un système de commande (33) configuré pour recevoir le signal de fuite de sang (BLS) et **caractérisé en ce que** le système de commande est en outre configuré pour émettre un signal qui, en coopération avec l'appareil de traitement du sang (1000), exécute une étape ou au moins une étape du groupe consistant à:
- émettre un avertissement à l'utilisateur qu'un nettoyage du troisième et/ou du quatrième connecteur/s (15, 17) est nécessaire;
- déclencher un nettoyage requis ou prévu du troisième et/ou du quatrième connecteur/s (15, 17);
- empêcher l'utilisation de l'appareil de traitement du sang (1000) jusqu'à ce que l'utilisateur ait exécuté au moins une activité requise; et
- empêcher de façon mécanique la connexion du troisième et/ou du quatrième connecteur/s (15, 17) avec le premier et/ou le second connecteur/s (3, 7);
lorsqu'un signal de fuite de sang (BLS) a été émis et/ou reçu.

2. L'appareil de traitement du sang (1000) selon la première revendication, comprenant en outre un appareil de surveillance (31), qui est configuré pour détecter une connexion du troisième et/ou du quatrième connecteur/s (15, 17) et qui est de plus configuré pour émettre et/ou recevoir un signal de connexion (VS) si une connexion est détectée, où le système de commande (33) est configuré pour initier au moins une étape du groupe, lorsqu'un signal de fuite de sang (BLS) ainsi qu'un signal de connexion (VS) ont été émis et/ou reçus.

3. L'appareil de traitement du sang (1000) selon la revendication 2, où l'appareil de surveillance comprend, ou consiste en, un capteur de connexion configuré et agencé pour détecter une connexion établie avec le troisième et/ou le quatrième connecteur/s (15, 17), et où l'appareil de surveillance est configuré pour émettre un signal de connexion (VS) si une connexion est détectée.

4. L'appareil de traitement du sang (1000) selon l'une quelconque des revendications précédentes, où l'appareil de traitement du sang (1000) comprend, ou est, un appareil interrogateur, au moyen duquel, en réponse à une interrogation, l'utilisateur peut ou doit confirmer et/ou infirmer une connexion établie, lorsqu'un signal de fuite de sang (BLS) a été émis et/ou reçu.

5. L'appareil de traitement du sang (1000) selon la revendication 4 où, en cas de confirmation, le traitement d'un patient au moyen de l'appareil de traitement du sang (1000) ne peut pas être poursuivi ou repris, ou un avertissement est émis indiquant d'interrompre le traitement ou de ne pas le recommencer.

6. L'appareil de traitement du sang (1000) selon l'une quelconque des revendications précédentes, où l'action requise est sélectionnée à partir d'un groupe constitué d'une saisie et d'une exécution d'étapes de nettoyage par l'utilisateur.

7. L'appareil de traitement du sang (1000) selon l'une quelconque des revendications précédentes, où l'appareil de traitement du sang (1000) comprend en outre un verrou ou un capot (35) qui, en état de verrouillage ou de recouvrement, recouvre au moins l'un du troisième et du quatrième connecteur (15, 17) dans un état de recouvrement de telle sorte qu'une connexion d'au moins un connecteur (15, 17) verrouillé ou recouvert avec le premier ou le second connecteur (3, 7) n'est pas possible, et qui peut être transféré dans un état de connexion dans lequel une connexion est possible,
où le fait d'empêcher de façon mécanique une connexion du troisième et/ou du quatrième connecteur/s (15, 17) avec le premier et/ou le second connecteur/s (3, 7) consiste à transférer le verrou ou le capot (35) dans l'état de verrouillage ou de recouvrement ou à le maintenir dans celui-ci.

8. L'appareil de traitement du sang (1000) selon la revendication 7, où le capot (35) est un clapet.

9. L'appareil de traitement du sang (1000) selon l'une des revendications précédentes, comprenant un dispositif de mémorisation (37) configuré ou commandé pour mémoriser le signal de fuite de sang (BLS) pendant un laps de temps prédéterminé ou jusqu'à ce qu'un événement déterminé se produise.

10. Un procédé de fonctionnement d'un appareil de traitement du sang (1000), comprenant les étapes consistant à:
- fournir un appareil de traitement du sang (1000) comprenant:
- un filtre de dialyse (100) ou un réceptacle (9) pour un filtre de dialyse (100);
- un conduit d'alimentation en fluide de dialyse (1) ayant un premier connecteur (3) pour alimenter le filtre de dialyse (100) en fluide de dialyse;
- un conduit d'évacuation de dialysat (5) ayant un second connecteur (7) pour évacuer le dialysat du filtre de dialyse (100);
- un capteur de fuite de sang (11) pour détecter du sang dans ou sur le filtre de dialyse (100), dans le conduit d'alimentation en fluide de dialyse (1) et/ou dans le conduit d'évacuation de dialysat (5),
et pour émettre un signal de fuite de sang si du sang est détecté;
- un conduit court-circuit (13) pour relier le conduit d'alimentation en fluide de dialyse (1) et, ou au, conduit d'évacuation de dialysat (5) en communication fluidique, le conduit court-circuit (13) comprenant pour ce faire un troisième connecteur (15) ainsi qu'un quatrième connecteur (17) destinés à être reliés respectivement à l'un du premier et du second connecteur (3, 7);
- un système de commande (33);
où le procédé comprend en outre les étapes suivantes:
- détecter au moyen d'un capteur de fuite de sang (11), s'il y a une fuite de sang et, le cas échéant, émettre un signal de fuite de sang (BLS);
- vérifier si un signal de fuite de sang (BLS) a été émis et/ou reçu et/ou traité;
- émettre un signal via le système d commande (33) qui, en coopération avec l'appareil de traitement du sang (1000), lorsqu'un signal de fuite de sang (BLS) a été émis et/ou reçu et/ou traité, exécute une étape ou au moins une étape du groupe, comprenant les étapes consistant à:
- émettre un avertissement à l'utilisateur qu'un nettoyage du troisième et/ou du quatrième connecteur/s (15, 17) est nécessaire;
- déclencher un nettoyage requis ou prévu du troisième et/ou du quatrième connecteur/s (15, 17);
- empêcher l'utilisation de l'appareil de traitement du sang (1000) jusqu'à ce que l'utilisateur ait exécuté au moins une activité requise; et
- empêcher de façon mécanique la connexion du troisième et/ou du quatrième connecteur/s (15, 17) avec le premier et/ou le second connecteur/s (3, 7).

11. Le procédé selon la revendication 10, où l'appareil de traitement du sang (1000) comprend un appareil de surveillance (31) pour détecter une connexion du troisième et/ou du quatrième connecteur/s (15, 17) et pour émettre un signal de connexion (VS);
et où le procédé comprend en outre l'étape consistant à:
- détecter au moyen de l'appareil de surveillance (31), si, après l'émission du signal de fuite de sang (BLS), le troisième et/ou le quatrième connecteur/s (15, 17) a/ont été relié/s et, le cas échéant, émettre un signal de connexion (VS);
où le système de commande (33) est configuré pour initier au moins une étape du groupe lorsqu'un signal de fuite de sang (BLS) ainsi qu'un signal de connexion (VS) ont été émis et/ou reçus et/ou traités.

12. Procédé non-thérapeutique pour changer un filtre de dialyse (100) d'un appareil de traitement du sang (1000), comprenant les étapes consistant à:
- fournir un appareil de traitement du sang (1000) comprenant:
- un premier filtre de dialyse (100) dans un réceptacle (9) pour filtre de dialyse (100);
- un conduit d'alimentation en fluide de dialyse (1) ayant un premier connecteur (3) pour alimenter le premier filtre de dialyse (100) en fluide de dialyse;
- un conduit d'évacuation de dialysat (5) ayant un second connecteur (7) pour évacuer le dialysat du premier filtre de dialyse (100);
- un capteur de fuite de sang (11) pour détecter du sang dans ou sur le filtre de dialyse (100), dans le conduit d'alimentation en fluide de dialyse (1) et/ou dans le conduit d'évacuation de dialysat (5),
et pour émettre un signal de fuite de sang si du sang est détecté;
- un conduit court-circuit (13) pour relier le conduit d'alimentation en fluide de dialyse (1) et, ou au, conduit d'évacuation de dialysat (5) en communication fluidique, le conduit court-circuit (13) comprenant pour ce faire un troisième connecteur (15) ainsi qu'un quatrième connecteur (17) destinés à être reliés respectivement à l'un du premier et du second connecteur (3, 7);
- un système de commande (33) configuré pour recevoir le signal de fuite de sang (BLS) et pour émettre un signal via le système de commande (33), qui, en coopération avec l'appareil de traitement du sang (1000), exécute une étape ou au moins une étape du groupe, consistant à:
- émettre un avertissement à l'utilisateur qu'un nettoyage du troisième et/ou du quatrième connecteur/s (15, 17) est nécessaire;
- déclencher un nettoyage requis ou prévu du troisième et/ou du quatrième connecteur/s (15, 17);
- empêcher l'utilisation de l'appareil de traitement du sang (1000), jusqu'à ce que l'utilisateur ait exécuté au moins une activité requise; et
- empêcher de façon mécanique la connexion du troisième et/ou du quatrième connecteur/s (15, 17) avec le premier et/ou le second connecteur/s (3, 7);
quand un signal de fuite de sang (BLS) a été émis et/ou reçu,
où le procédé comprend en outre les étapes suivantes:
- fournir un second filtre de dialyse (100);
- séparer le conduit d'alimentation en fluide de dialyse (1) et le conduit d'évacuation de dialysat (5) du premier filtre de dialyse (100);
- relier le conduit d'alimentation en fluide de dialyse (1) et le conduit d'évacuation de dialysat (5) au second filtre de dialyse (100), sans avoir préalablement relié le conduit d'alimentation en fluide de dialyse (1) ou le conduit d'évacuation de dialysat (5) au conduit court-circuit (13).
